# FASCICULE DE BREVET EUROPEEN

(11) **EP 0 629 612 B1**
(45) Date de publication et mention de la délivrance du brevet: **01.12.1999**
(21) Numéro de dépôt: 94201643.7
(22) Date de dépôt: 09.06.1994
(51) Int. Cl.: C07C 273/18, C07D 209/20, C07C 323/59, C07D 243/04, C07D 239/10, C07K 5/08

(54) **Procédé de préparation d'uréines dérivées d'alpha,omega-diaminoacides**
Verfahren zur Herstellung von alpha,omega-Diaminosäuren abgeleiteten Harnstoffen
Process for the preparation of ureas derived from alpha, omega-diaminoacids

(30) Priorité: 18.06.1993 BE 9300621
(43) Date de publication de la demande: 21.12.1994
(60) Demande divisionnaire: 99103523.9
(73) Titulaire: SOLVAY (Société Anonyme), 1050 Bruxelles (BE)
(72) Inventeur: Callens, Roland, B-9031 Drongen (BE); Blondeel, Georges, B-9300 Aalst (BE); Anteunis, Marc, B-9030 Mariakerke (BE); Becu, Frank, B-8490 Jabbeke (BE)
(74) Mandataire: Jacques, Philippe

(56) Documents cités:
- EP-A- 0 074 070
- EP-A- 0 207 358
- US-A- 2 288 422
- US-A- 5 032 577
- JOURNAL OF ORGANIC CHEMISTRY, vol. 48, no. 12 , 17 Juin 1983 Washington, DC, US, pages 2014 - 2021 J.T. CAPECCHI, ET AL.: 'Critical examination of a method for the analysis of alpha and omega linkages in peptides containing aspartic acid and glutamic acid'
- INTERNATIONAL JOURNAL OF PEPTIDE AND PROTEIN RESEARCH vol. 33, no. 6 , 1989 Copenhague, DK, pages 403 - 411 H. MAEDA, ET AL.: 'Synthesis and central nervous system actions of thyrotropin- releasing hormone analogues containing a 1-substituted 2-oxoimidazolidine moiety'
- JOURNAL OF MEDICINAL CHEMISTRY, vol. 33, no. 8 , Août 1990 Washington, DC, US, pages 2130 - 2137 M. SUZUKI, ET AL.: 'Synthesis and central nervous system actions of thyrotropin- releasing hormone analogues containing a dihydroorotic acid moiety'

## Description

La présente invention se rapporte à un procédé de préparation d'uréines, dérivées d'α,ω-diaminoacides.

Classiquement, des uréines dérivées de diaminoacides peuvent être préparées par carbamoylation au moyen d'un cyanate ou d'un isocyanate. Cette procédure connue n'apparaît cependant pas entièrement satisfaisante, principalement en raison d'une spécificité insuffisante de ces réactifs pour la fonction amino à carbamoyler et en raison de la racémisation parfois importante qu'un tel traitement peut provoquer.

L'invention remédie aux inconvénients des procédés classiques en fournissant un nouveau procédé particulièrement performant, permettant d'obtenir le produit désiré avec un rendement chimique amélioré, en conservant remarquablement la pureté chirale des composés mis en oeuvre.

En conséquence, l'invention concerne un procédé de préparation d'uréines dérivées d'un α,ω-diaminoacide, selon lequel on fait réagir, en milieu basique, un composé contenant un groupement amino libre avec un dérivé du diaminoacide comportant un groupement N^{ω}-aryloxycarbonyle.

Par uréine, on entend désigner tout composé dont la structure moléculaire contient la structure -NH-CO-NH-.

Par aminoacide, on entend désigner, aux fins de la présente invention, tout composé comprenant au moins un groupement amino et au moins un groupement carboxyle. Par extension, on entend aussi englober ci-après sous le vocable "aminoacide", tout aminoacide dont certains autres groupements sont éventuellement liés à des groupements organiques tels que des groupements protecteurs. En particulier, par α,ω-diaminoacide, on entend désigner tout aminoacide comprenant au moins un groupement amino et au moins un groupement carboxyle liés au même atome de carbone de la molécule et comprenant en outre au moins un autre groupement amino lié à un autre atome de carbone. Il s'agit le plus souvent d'un composé de formule générale 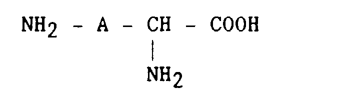 dans laquelle A représente un groupement bivalent, constitué d'une chaîne carbonée de 1 à 8 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto, sans que le nombre total d'atomes de carbone dans le groupement A ne soit supérieur à 15. De préférence, A est un groupement polyméthylène comprenant de 2 à 5 atomes de carbone. A titre d'exemples d'α,ω-diaminoacides, on peut citer notamment l'acide 2,3-diaminopropanoïque, l'acide 2,4-diaminobutanoïque, l'ornithine, la lysine, l'homolysine, la 5-hydroxylysine, la 6-méthyllysine et l'acide 2,6-diaminopimélique.

Par dérivé du diaminoacide comportant un groupement N^{ω}-aryloxycarbonyle, également dénommé par la suite N^{ω}-aryloxycarbonyl-diaminoacide, on entend désigner tout dérivé de α,ω-diaminoacide dans lequel un groupement aryloxycarbonyle de formule R-O-CO-, R symbolisant un groupement aryle, est lié à l'atome d'azote du groupement ω-amino du diaminoacide.

Dans le procédé selon l'invention, la mise en oeuvre d'un dérivé du diaminoacide comportant un groupement N^{ω}-aryloxycarbonyle est critique. En effet, il est apparu de manière surprenante que le groupement aryloxycarbonyle lié au groupement ω-amino de l'aminoacide conduit, en présence d'un composé contenant un groupement amino libre, à la formation d'une uréine, par substitution du fragment aryloxy dudit groupement aryloxycarbonyle par le groupement amino libre dudit composé.

Généralement, le dérivé N^{ω}-aryloxycarbonyle du diaminoacide mis en oeuvre comporte, comme groupement aryloxycarbonyle, un groupement comprenant de 7 à 15 atomes de carbone. Le plus souvent, ce groupement aryloxycarbonyle est un groupement phényloxycarbonyle ou naphtyloxycarbonyle, éventuellement substitué par au moins un groupement choisi parmi les groupements alkyles comprenant de 1 à 4 atomes de carbone et le groupement nitro. A titre d'exemples de groupements aryloxycarbonyles utilisables dans le procédé selon l'invention, on peut citer les groupements phényloxycarbonyle, tolyloxycarbonyle, xylyloxycarbonyle, mésitylyloxycarbonyle, éthylphényloxycarbonyle, diéthylphényloxycarbonyle, propylphényloxycarbonyle, isopropylphényloxycarbonyle, naphtyloxycarbonyle et nitrophényloxycarbonyle. De préférence, le groupement aryloxycarbonyle est un groupement phényloxycarbonyle ou p-tolyloxycarbonyle. De bons résultats ont été obtenus dans le procédé selon l'invention avec le dérivé N^{ω} -phényloxycarbonyle du diaminoacide.

Un dérivé N^{ω}-aryloxycarbonyle de n'importe quel α,ω-diaminoacide peut être mis en oeuvre dans le procédé selon l'invention.

Le N^{ω}-aryloxycarbonyl-diaminoacide est un produit facilement accessible et peu coûteux. Il peut être préparé classiquement en recourant à des techniques bien connues d'acylation sélective, par exemple par l'intermédiaire du complexe cuivrique selon une procédure similaire à celle décrite notamment dans "Methoden Der Organischen Chemie" (HOUBEN-WEYL), 1974, Volume XV/1, p. 472, au sujet de la N^{ε}-benzyloxycarbonyl-L-lysine. La fonction α-amino étant complexée par l'ion cuivre, le groupement aryloxycarbonyle peut être fixé sélectivement à la fonction ω-amino du diaminoacide par réaction avec un chloroformiate d'aryle ou un aryloxycarbonyloxysuccinimide.

Le composé comprenant un groupement amino libre qui réagit avec le N^{ω} -aryloxycarbonyl-diaminoacide dans le procédé selon l'invention est tout composé de formule générale R1R2NH dans laquelle R1 et R2 représentent indépendamment l'un de l'autre, des atomes d'hydrogène, des radicaux alkyles, cycloalkyles ou aralkyles ou dans laquelle R1 et R2 forment ensemble un radical alicyclique. Dans ce composé, les radicaux alkyles, cycloalkyles, aralkyles ou alicycliques peuvent être substitués par un ou plusieurs groupements fonctionnels comprenant au moins un atome d'oxygène, de soufre ou d'azote, par exemple par un groupement carboxyle, hydroxyle, mercapto, indolyle ou imidazolyle. Des composés utilisables dans le procédé selon l'invention sont notamment l'ammoniac, les amines primaires ou secondaires et les aminoacides tels que définis plus haut. Le procédé selon l'invention est particulièrement intéressant lorsque le composé comprenant un groupement amino libre est un aminoacide.

Lorsque le N^{ω}-aryloxycarbonyl-diaminoacide est un dérivé d'un α,ω-diaminoacide dans lequel la chaîne carbonée du groupement A est constituée de 1 à 3 atomes de carbone, le N^{ω}-aryloxycarbonyl-diaminoacide joue, dans le procédé selon l'invention, à la fois le rôle de dérivé N^{ω}-aryloxycarbonyle et, par son groupement α-amino, le rôle de composé contenant un groupement amino libre. Il en résulte, via une réaction intramoléculaire, la formation d'uréines cycliques de formule générale 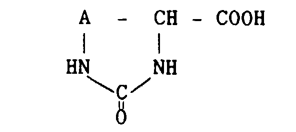 dans laquelle A représente un groupement bivalent, constitué d'une chaîne carbonée linéaire formée de 1 à 3 atomes de carbone, éventuellement substituée.

A titre illustratif, dans le procédé de l'invention, l'acide N^{β}-aryloxycarbonyl-2,3-diaminopropanoïque forme l'acide 2-oxo-imidazolidine-4-carboxylique, l'acide N^{γ}-aryloxycarbonyl-2,4-diaminobutyrique forme l'acide 2-oxo-hexahydropyrimidine-4-carboxylique et la N^{δ}-aryloxycarbonyl-ornithine forme l'acide 2-oxo-hexahydro-1,3-diazépine-4-carboxylique.

Lorsque le N^{ω}-aryloxycarbonyl-diaminoacide est un dérivé d'un α,ω-diaminoacide dans lequel la chaîne carbonée du groupement A est constituée d'au moins 4 atomes de carbone, le N^{ω}-aryloxycarbonyl-diaminoacide est transformé dans le procédé selon l'invention en uréine non cyclique de formule générale 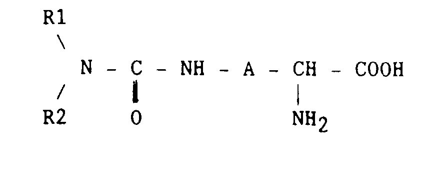 dans laquelle R1 et R2 ont la même signification que ci-avant et dans laquelle A représente un groupement bivalent, constitué d'une chaîne carbonée linéaire formée d'au moins 4 atomes de carbone, éventuellement substituée. A titre illustratif, dans le procédé de l'invention, l'homocitrulline est obtenue par réaction entre la N^{ε}-phényloxycarbonyl-lysine et l'ammoniac. Lorsque le composé comprenant un groupement amino libre est un aminoacide, un N^{ω}-(aminoacidocarbonyl)-α,ω-diaminoacide, également dénommé N^{ω}-(carboxyalkylcarbamoyl)-α,ω-diaminoacide, est obtenu par le procédé selon l'invention.

Un N^{ω}-aryloxycarbonyl-diaminoacide intégré au sein d'une chaîne peptidique peut sans inconvénient être mis en oeuvre dans le procédé selon l'invention. En particulier, lorsque le N^{ω}-aryloxycarbonyl-diaminoacide est un dérivé d'un α,ω-diaminoacide dans lequel la chaîne carbonée du groupement A est constituée de 1 à 3 atomes de carbone et que ce composé constitue le résidu N-terminal d'une chaîne peptidique, le procédé selon l'invention permet d'obtenir facilement des peptides de formule générale 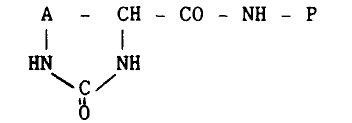 dans laquelle A représente un groupement bivalent, constitué d'une chaîne carbonée linéaire formée de 1 à 3 atomes de carbone, éventuellement substituée et dans laquelle NH - P représente toute chaîne peptidique liée à l'uréine cyclique par une liaison amide.

Le procédé selon l'invention est effectué dans un milieu basique.

Le procédé selon l'invention est généralement effectué dans un milieu liquide dans lequel le N^{ω}-aryloxycarbonyl-diaminoacide et le composé comprenant un groupement amino libre sont au moins en partie solubles et de préférence entièrement solubles. Selon la nature des réactifs, le milieu peut comprendre de l'eau et/ou un solvant organique. Des solvants organiques qui conviennent dans le procédé selon l'invention sont les alcools inférieurs tels que notamment le méthanol, l'éthanol et l'isopropanol, le tétrahydrofurane et le diméthoxyéthane. Les milieux constitués d'eau et d'un solvant organique miscible à l'eau sont préférés. De bons résultats ont été obtenus notamment dans un milieu eau-éthanol.

La basicité du milieu peut être obtenue par ajout d'un composé basique dans le milieu, par exemple par ajout d'une base inorganique telle que LiOH, NaOH ou KOH ou par ajout d'une base organique inerte dans les conditions de réaction, telle qu'une amine tertiaire. De bons résultats ont par exemple été obtenus en présence de LiOH ou de triéthylamine. Lorsque le composé contenant un groupement amino libre est un aminoacide comportant des fonctions carboxyles libres, le composé basique doit être mis en oeuvre en quantité suffisante pour neutraliser les fonctions carboxyles.

Pour l'obtention des uréines cycliques, la réactivité intramoléculaire du N^{ω} -aryloxycarbonyl-diaminoacide est telle que l'on peut sans problème ajouter un composé contenant un groupement amino libre, tel que l'ammoniac, pour atteindre la basicité souhaitée du milieu, sans que cela n'affecte de manière appréciable le rendement de la réaction en uréine cyclique.

Pour l'obtention des uréines non cycliques, lorsque le groupement α-amino du N^{ω}-aryloxycarbonyl-diaminoacide est libre, il est nécessaire, afin d'éviter une réaction de condensation du N^{ω}-aryloxycarbonyl-diaminoacide, concurrente à la réaction désirée avec le composé contenant un groupement amino libre, de travailler avec un excès du composé comportant un groupement amino libre que l'on désire faire réagir avec le N^{ω}-aryloxycarbonyl-diaminoacide, par rapport à la quantité stoechiométrique nécessaire. De bons résultats sont obtenus lorsqu'on travaille avec un rapport molaire entre le composé contenant un groupement amino libre et le N^{ω}-aryloxycarbonyl-diaminoacide au moins égal à 3. De préférence, on travaille avec un rapport au moins égal à 4. En principe, il n'y a pas de limite supérieure à ce rapport. En pratique, il est cependant généralement inutile de travailler avec un rapport molaire entre le composé contenant un groupement amino libre et le N^{ω}-aryloxycarbonyl-diaminoacide supérieur à 100. Le plus souvent, le rapport molaire ne dépasse pas 10. Lorsque le composé contenant un groupement amino libre est un aminoacide ou un peptide, le rapport molaire ne dépasse pas, de préférence, 7. Lorsque le composé contenant un groupement amino libre présente un caractère basique suffisant, il peut s'avérer inutile de rajouter un autre composé basique dans le milieu.

Le procédé selon l'invention peut être mis en oeuvre dans une large gamme de concentration des réactifs dans le milieu liquide, en particulier pour l'obtention des uréines cycliques. En général, le N^{ω}-aryloxycarbonyl-diaminoacide est mis en oeuvre à une concentration de 0,05 à 5 mole/l, de préférence de 0,1 à 1 mole/l.

La réaction peut être effectuée de la température ambiante jusqu'à la température d'ébullition du solvant organique. Elle est avantageusement effectuée de 30 à 80 °C. Une température de 40 à 60 °C est tout particulièrement préférée.

Dans ces conditions, le temps de réaction est généralement inférieur à 10 heures. Le plus souvent, la réaction est complète après une durée de 30 minutes à 4 heures.

Le procédé selon l'invention apparaît particulièrement avantageux pour préparer des uréines dérivées d'α,ω-diaminoacides. Le dérivé N^{ω}-aryloxycarbonyle du diaminoacide mis en oeuvre dans le procédé selon l'invention peut être préparé facilement et à peu de frais au départ du diaminoacide. Il peut être aisément isolé sous forme pure. Il est stable et peut être conservé longtemps sans dégradation. Le procédé selon l'invention est particulièrement performant. Il permet d'obtenir les uréines recherchées avec un rendement très élevé. Il est en outre très respectueux de la chiralité des composés mis en oeuvre. De plus, le départ du fragment aryloxy du groupement aryloxycarbonyle ne génère dans le milieu que des sous-produits relativement inoffensifs qui ne perturbent pas la synthèse. Par exemple, lorsqu'il s'agit du groupement phényloxycarbonyle, seuls du phénol est généré. Dès lors, lorsque les composés mis en oeuvre comportent des groupements très labiles, tels que certains groupements protecteurs, les sous-produits générés dans le milieu ne provoquent aucune dégradation de ces composés. La purification des produits désirés est nettement plus simple que dans les procédés antérieurs connus.

L'invention concerne la préparation de N^{ω}-(aminoacidocarbonyl)-α,ω-diaminoacides, uréines dérivées d'un α,ω-diaminoacide, de formule générale 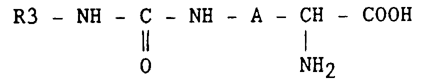 dans laquelle A représente un groupement bivalent, constitué d'une chaîne carbonée linéaire formée de 4 à 8 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto et dans laquelle R3 - NH représente un aminoacide ou un peptide. De préférence A est un groupement polyméthylène contenant 4 ou 5 atomes de carbone. R3 - NH est de préférence un aminoacide et, plus préférentiellement, un aminoacide essentiel.

Ces composés nouveaux constituent des composés de structure proche de celle de dipeptides et sont utilisables notamment en lieu et place des dipeptides correspondants, notamment comme source d'aminoacides essentiels en alimentation humaine parentérale ou en alimentation animale.

L'invention concerne également la préparation d'uréines cycliques de formule générale 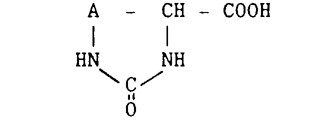 dans laquelle A représente un groupement bivalent, constitué d'une chaîne carbonée linéaire formée de 1 à 3 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto, à l'exclusion de l'acide 2-oxo-imidazolidine-4-carboxylique et de l'acide (LD)-2-oxo-hexahydropyrimidine-4-carboxylique. De préférence, A représente un groupement bivalent, constitué d'une chaîne carbonée constituée de 2 ou 3 atomes de carbone, éventuellement substituée. De manière particulièrement préférée, A représente un groupement triméthylène -(CH₂)₃-. Dans ce cas, l'uréine formée est l'acide 2-oxo-hexahydro-1,3-diazépine-4-carboxylique, obtenu aisément par le procédé selon l'invention au départ de l'ornithine.

Selon que l'énantiomère (D) ou (L) du diaminoacide est mis en oeuvre dans le procédé selon l'invention, l'énantiomère (D) ou (L) de l'uréine cyclique correspondante est obtenu sous forme chiralement pure.

Ces uréines cycliques peuvent être utilisées notamment comme résidu N-terminal de certains peptides biologiquement actifs, tels l'hormone TRH (Thyrotropin Releasing Hormone), en remplacement du groupement pyroglutamyl N-terminal de ce peptide.

Il s'agit de peptides analogues de la TRH, de formule générale 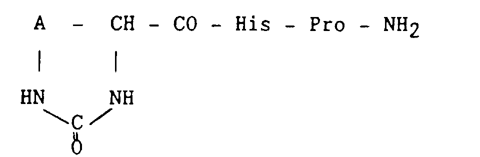 dans laquelle A est un groupement bivalent, constitué d'une chaîne carbonée linéaire formée de 2 ou 3 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto. De préférence, A est un groupement polyméthylène. Le peptide dans lequel A est un groupement triméthylène est préféré. Ces peptides présentent une résistance accrue à la digestion protéolytique, tout en conservant une activité biologique élevée.

Les représentations symboliques des aminoacides et des peptides adoptées dans la description et les exemples suivent les recommandations IUPAC de nomenclature, généralement adoptées et décrites par exemple dans "Nomenclature and Symbolism for Amino Acids and Peptides, Recommendations 1983", Eur. J. Biochem. (1984), 138, p. 9-37. Sauf stipulation contraire, tous les aminoacides décrits sont les (L)-aminoacides.

Les exemples suivants illustrent l'invention.

Les différents produits et intermédiaires de synthèse rapportés dans les exemples ont été caractérisés par différentes méthodes analytiques, mises en oeuvre dans les conditions suivantes :
- rotation optique (α) : mesurée à 589 nm à 25 °C
- Chromatographie en couche mince (CCM) :
   . Plaques de silicagel MERCK 60F-254
   . éluants :
      Rf(1) Acétate d'éthyle:n-butanol:acide acétique:eau 1:1:1:1
      Rf(2) Acétonitrile:chloroforme:acide acétique:eau 5:2:2:1
      Rf(3) Acétonitrile:chloroforme:acide acétique:eau 7:4:4:2
- Chromatographie HPLC :
   . Colonne C-18 Vydac 5µm
   . Elution : gradient de 98 % A + 2 % B jusqu'à 25 % A + 75 % B en 49 minutes (A = eau 0,1 % en acide trifluoroacétique ; B = acétonitrile 0,1 % en acide trifluoroacétique)
   . Débit = 2 ml/min
   . Détection : UV 220 nm.
- Résonance magnétique nucléaire (RMN) :
   . Appareil Brüker AMX 500 MHz
   . Shift donnés en ppm
   . Allures des résonances : m=multiplet, s=singulet, d=doublet, t=triplet, q=quadruplet, quint=quintuplet, o=octuplet.

### Exemple 1 : Synthèse de N^{ε}-(N^{α}-tryptophanocarbonyl)-lysine

5,1 g (25 mmol) de tryptophane, 1,34 g (5 mmol) de N^{ε}-phényloxycarbonyl-lysine et 1,05 g (25 mmol) de LiOH.H₂O ont été pesés dans un ballon de 100 ml, puis 40 ml d'eau ont été introduits dans le ballon. Celui-ci a été immergé dans un bain d'huile maintenu à 75°C pendant 45 minutes, a ensuite été rapidement refroidi à température ambiante sous de l'eau courante, puis traité avec 25 ml d'acide chlorhydrique. Le précipité formé a été filtré, après une nuit au réfrigérateur.

Une analyse HPLC a révélé une conversion complète de la N^{ε}-phényloxycarbonyl-lysine en 2 produits présentant, dans les conditions d'analyse, un temps de rétention (tR) de 12,23 et 13,95 minutes, avec un rapport 13:1 en surface de pics. Les produits ont été séparés par injection du filtrat tel quel sur une colonne HPLC C-18 préparative, puis lyophilisés. 880 mg de N^{ε}-(N^{α}-tryptophanocarbonyl)-lysine et 76 mg de N^{ε}-(N^{ε}-(N^{α}-tryptophanocarbonyl)-N^{α}-lysinocarbonyl)-lysine ont été obtenus. Les propriétés physico-chimiques de la N^{ε}-(N^{α}-tryptophanocarbonyl)-lysine sont les suivantes :
PF : 133°C
α : + 3,31 (c = 1, 1 % acide acétique)
CCM : Rf(3) = 0,34

| RMN (H-1) dans le DMS0-d6 : | |
|---|---|
| 11,00 (1H s) NH indole | 7,50 (1H d) H4 indole |
| 7,32 (1H d) H7 indole | 7,09 (1H s) H2 indole |
| 7,03 (1H t) H6 indole | 6,94 (1H t) H5 indole |
| 6,28 (1H t large) εNH Lys | 6,18 (1H d) αNH Trp |
| 4,36 (1H m) Hα Trp | 3,30 (1H m) Hα Lys |
| 3,12 (1H dd) HβATrp | 2,99 (1H dd) HβBTrp |
| 2,94 (2H m) Hε's Lys | 1,70 (1H m) HβA Lys |
| 1,60 (1H m) HβB Lys | 1,31 (4H m) Hγ's + Hδ's Lys |

### Exemple 2 : Synthèse de N^{ε}-(méthioninocarbonyl)-lysine

La N^{ε}-(méthioninocarbonyl)-lysine a été préparée au départ de méthionine et de N^{ε}-phényloxycarbonyl-lysine selon la même recette que celle décrite à l'exemple 1. Elle présente les propriétés physico-chimiques suivantes :
- PF :: 148°C
- α :: - 9,5 (c = 1, 1 % acide acétique)
- CCM :: Rf(3) = 0,27
- HPLC :: tR = 7,14 min

| RMN (H-1) dans le DMS0-d6 : | |
|---|---|
| 6,43 (1H d large) NHα Met | 6,33 (1H t large) NHε Lys |
| 4,11 (1H m) Hα Met | 3,35 (1H m) Hα Lys |
| 2,95 (2H m) Hε's Lys | 2,33 (2H t) Hγ's Met |
| 2,02 (3H s) CH3 Met | 1,88 (1H m) HβA Met |
| 1,77 (1H m) HβB Met | 1,72 (1H m) HβA Lys |
| 1,65 (1H m) HβB Lys | 1,33 (4H m) Hγ's + Hδ's Lys |

### Exemple 3 : synthèse de l'acide (D)-2-oxo-hexahydro-1,3-diazépine-4-carboxylique

A une suspension de 1,27 g (5 mmol) de (D)-N^{δ}-phényloxycarbonyl-ornithine dans 15 ml de diméthoxyéthane et 10 ml d'eau ont été ajoutés 7,7 ml (± 100 mmol) d'ammoniaque à 25 %. Le degré de conversion a été suivi par CCM. Une fois la (D)-phényloxycarbonyl-ornithine disparue, le milieu réactionnel a été concentré à sec. Le résidu a été trituré par 20 ml d'acétone à 80 %, filtré et séché. A ce stade, on a déterminé par RMN que le produit brut obtenu était le sel d'ammonium de l'acide (D)-2-oxo-hexahydro-1,3-diazépine-4-carboxylique, contaminé par environ 5 % de citrulline (Rendement : 820 mg ou 92 % en produit brut). L'acide (D)-2-oxo-hexahydro-1,3-diazépine-4-carboxylique a été obtenu, avec une pureté supérieure à 98 %, par passage du produit brut en solution aqueuse à travers une colonne résine échangeuse d'ions sous forme H⁺, puis lyophilisation.
PF : 130-150 °C (décomposition)
α : + 14,6 (c = 1, eau)
CCM :
Rf(3) : 0,70 ne réagit plus à la ninhydrine
Rf(3) : 0,16 pour la citrulline
Rf(3) : 0,54 pour la N^{δ}-phényloxycarbonyl-ornithine

| RMN(H) réf DMS0-d6 à 2,49 : | |
|---|---|
| 6,20 (1H,s large) NH1 | 5,55 (1H,s large) NH3 |
| 3,75 (1H,m) H4 | 2,89 (2H,m) H7 |
| 1,90 (1H,m) H5A | 1,77 (1H,m) H5B |
| 1,55 (2H,m) H's 6 | |

| RMN(C-13) réf DMS0-d6 à 39,50 : | |
|---|---|
| 173,45 (COOH) | 163,65 (C2) |
| 54,43 (C4) | 31,03 (C5) |
| 26,81 (C6) | |

### Exemple 4 : Synthèse de l'acide (L)-2-oxo-hexahydropyrimidine-4-carboxylique

2 g (8,3 mmol) d'acide (L)-N^{γ}-phényloxycarbonyl-diaminobutyrique ont été dissous dans 10 ml d'eau et 5 ml de méthanol. Après ajout de 3 ml (22 mmol) de triéthylamine, la solution a été chauffée sous léger reflux jusqu'à disparition complète du produit de départ (contrôle par CCM). La solution a été concentrée à sec, puis suspendue dans 50 ml de dichlorométhane. Le produit alors sous la forme (L)-2-oxo-hexahydropyrimidine-4-carboxylate de triéthylamine a été déplacé par addition de 1,8 ml d'acide trifluoroacétique. Le trifluoroacétate de triéthylamine étant bien soluble, l'acide (L)-2-oxo-hexahydropyrimidine-4-carboxylique précipite sélectivement. Il a été récupéré par filtration, lavé par du dichlorométhane, puis séché.
Rendement : 95 Z
α : + 20,1 (c = 1, eau)
CCM Rf(2) = 0,55

| RMN(¹H) réf D20 à 4,80 : | |
|---|---|
| 4,27 (1H,t) Hα | 3,35 (1H d de t) HγA |
| 3,22 (1H o) HγB | 2,13 à 2,18 (2H m) HβA+B |

### Exemple 5 : Synthèse de (2-oxo-hexahydro-1,3-diazépine-4-carbonyl)-His-Pro-NH₂

1 g (1,40 mmol) de bis-trifluoroacétate de N^{δ}(phényloxycarbonyl)Orn-His-Pro-NH₂ a été dissous dans 10 ml de méthanol contenant 0,75 ml (5,4 mmol) de triéthylamine. La solution a été portée à léger reflux à une température d'environ 65 °C, jusqu'à disparition du produit de départ (contrôle par CCM). La solution a été concentrée à sec et triturée avec 20 ml de dichlorométhane. Après filtration, le produit brut recueilli (0,52 g) a été purifié par chromatographie préparative sur phase inverse C-4. L'échantillon analytique isolé sous forme de sel d'acétate présente les propriétés physico-chimiques suivantes :
α = -8,1 (c = 1, 1 % acide acétique)
PF : 105 °C
CCM Rf(1) = 0,42
RMN (¹H, D20) : certaines résonances sont dédoublées à cause de l'isomerie cis/trans au niveau de la liaison His-Pro. Les résultats pour la forme majeure (± 85 %) sont repris ci-après :

| | |
|---|---|
| 8,56 (1H s) H2 imidazole | 7,34 (1H s) H5 imidazole |
| 5,08 (1H dd) Hα His | 4,46 (1H dd) Hα Pro |
| 4,07 (1H dd) Hα 0dc | 3,81 (1H m) HδA Pro |
| 3,65 (1H m) HδB Pro | 3,30 (1H dd) HβA His |
| 3,19 (1H dd) HβB His | 3,08 à 3,02 (2H m) HδA+B Odc |
| 2,36 (1H m) HβA Pro | 2,15 à 1,95 (5H + CH3 acétate) |
| 1,74 (1H m) HγA 0dc | 1,51 (1H m) HγB Odc |

### Exemple 6 : Synthèse de (2-oxo-hexahydropyrimidine-4-carbonyl)-His-Pro-NH₂

432 mg (3 mmol) d'acide (L)-2-oxo-hexahydropyrimidine-4-carboxylique ont été dissous dans 10 ml de N-méthylpyrrolidone contenant 0,33 ml (3 mmol) de N-méthylmorpholine. A la solution refroidie à -10°C, ont été ajoutés 0,39 ml de chloroformiate d'isobutyle. Après 5 minutes de réaction à -10°C. une solution de 5 ml de N-méthylpyrrolidone contenant 1,25 g (3 mmol) de 2HBr.His-Pro-NH₂ et 0,90 ml de triéthylamine (6,5 mmol) a été ajoutée. Après un temps de mûrissage de 30 minutes à température ambiante, le mélange réactionnel a été ajouté goutte-à-goutte à 50 ml d'éther sulfurique. Le précipité obtenu a été filtré, puis lavé 2 fois avec 20 ml de dichlorométhane et séché, donnant 1,3 g de produit. Il a été purifié par passage sur une colonne de silicagel en utilisant la même phase mobile qu'en CCM.

Données analytiques :
α = -23,8 (c = 1, acide acétique)
PF = 140 °C
CCM: Rf(1) = 0,33

RMN(¹H) dans le D20. Certaines résonances sont dédoublées à cause de l'isomerie cis/trans au niveau de la liaison His-Pro. Les résultats pour la forme majeure (± 85 %) sont repris ci-après :

| | |
|---|---|
| 8,16 (1H s) H2 imidazole | 7,21 (1H s) H5 imidazole |
| 5,04 (1H dd) Hα His | 4,48 (1H dd) Hα Pro |
| 4,17 (1H s large) Hα 0pc | 3,88 (1H m) HδA Pro |
| 3,68 (1H m) HδB Pro | 3,30 (2H d large) HβA His + HγA 0pc |
| 3,14 (1H dd) HβB His | 2,78 (1H m) HγB 0pc |
| 2,37 (1H m) HβA Pro | 2,20 à 2,00 HβB + Hγ's Pro/Hβ's 0pc |

## Revendications

1. Procédé de préparation d'uréines dérivées d'un α,ω-diaminoacide selon lequel on fait réagir, en milieu basique, un composé contenant un groupement amino libre avec un dérivé du diaminoacide comportant un groupement N^{ω}-aryloxycarbonyle.

2. Procédé selon la revendication 1, caractérisé en ce que le dérivé du diaminoacide mis en oeuvre comporte, comme groupement aryloxycarbonyle, un groupement comprenant de 7 à 15 atomes de carbone.

3. Procédé selon la revendication 2, caractérisé en ce que le groupement aryloxycarbonyle est un groupement phényloxycarbonyle ou naphtyloxycarbonyle, éventuellement substitué par au moins un groupement choisi parmi les groupements alkyles comprenant de 1 à 4 atomes de carbone et le groupement nitro.

4. Procédé selon la revendication 3, caractérisé en ce que le groupement aryloxycarbonyle est le groupement phényloxycarbonyle.

5. Procédé selon une quelconque des revendications 1 à 4, caractérisé en ce que le composé comprenant un groupement amino libre est choisi parmi l'ammoniac, les amines primaires et secondaires et les aminoacides.

6. Procédé selon la revendication 5, caractérisé en ce que le composé comprenant un groupement amino libre est un aminoacide.

7. Procédé selon la revendication 6, appliqué à la préparation de N^{ω}-(aminoacidocarbonyl)-α,ω-diaminoacides de formule générale 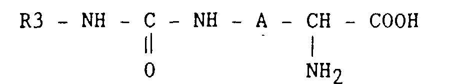 dans laquelle A représente un groupement bivalent, constitué d'une chaîne carbonée linéaire formée de 4 à 8 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto et dans laquelle R3 - NH représente un aminoacide ou un peptide.

8. Procédé selon la revendication 6, appliqué à la préparation d' uréines cycliques de formule générale 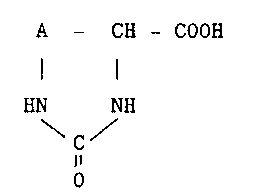 dans laquelle A représente un groupement bivalent, constitué d'une chaîne carbonée linéaire formée de 1 à 3 atomes de carbone, laquelle est éventuellement substituée par un ou plusieurs groupements choisis parmi les groupements alkyles en C1-C3 et les groupements fonctionnels comprenant au moins un atome d'oxygène ou de soufre tels qu'un groupement carboxyle, acyle, hydroxyle, alcoxyle ou mercapto, à l'exclusion de l'acide 2-oxo-imidazolidine-4-carboxylique et de l'acide (LD)-2-oxo-hexahydropyrimidine-4-carboxylique.

9. Procédé selon la revendication 8 appliqué à la préparation d'une uréine cyclique dans laquelle A représente un groupement triméthylène -(CH₂)₃-.

## Claims

1. Process for the preparation of ureins derived from an α,ω-diamino acid according to which a compound containing a free amino group is reacted, in basic medium, with a diamino acid derivative containing an N^{ω}-aryloxycarbonyl group.

2. Process according to Claim 1, characterized in that the diamino acid derivative used contains, as aryloxycarbonyl group, a group comprising from 7 to 15 carbon atoms.

3. Process according to Claim 2, characterized in that the aryloxycarbonyl group is a phenyloxycarbonyl or naphtyloxycarbonyl group optionally substituted by at least one group chosen from alkyl groups comprising from 1 to 4 carbon atoms and the nitro group.

4. Process according to Claim 3, characterized in that the aryloxycarbonyl group is the phenyloxycarbonyl group.

5. Process according to any one of the Claims 1 to 4, characterized in that the compound comprising a free amino group is chosen from ammonia, primary and secondary amines and amino acids.

6. Process according to Claim 5, characterized in that the compound comprizing a free amino group is an amino acid.

7. Process according to Claim 6, applied to the preparation of N^{ω} -carboxyalkylcarbamoyl-α,ω-diamino acids of general formula 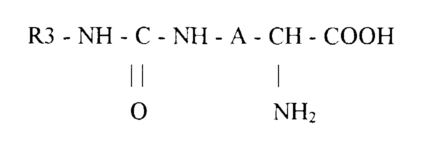 in which A represents a bivalent group consisting of a linear carbon chain formed from 4 to 8 carbon atoms, which chain is optionally substitued by one or a number of groups chosen from C₁-C₃ alkyl groups and functional groups comprising at least one oxygen or sulphur atom such as a carboxyl, acyl, hydroxyl, alkoxy or mercapto group, and in which R3-NH represents an amino acid or a peptide.

8. Process acording to Claim 6, applied to the preparation of cyclic ureins of general formula 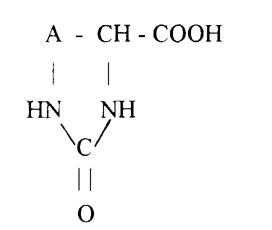 in which A represents a bivalent group consisting of a linear carbon chain formed from 1 to 3 carbon atoms, which chain is optionally substituted by one or a number of groups chosen from C₁-C₃ alkyl groups and functional groups comprising at least one oxygen or sulphur atom such as a carboxyl, acyl, hydroxyl, alkoxy or mercapto group, with the exception of 2-oxoimidazolidinyl-4-carboxylic acid and (LD)-2-oxohexahydropyrimidinyl-4-carboxylic acid.

9. Process according to Claim 8 applied to the preparation of a cyclic ureins in which A represents a trimethylene group -(CH₂)₃-.

## Patentansprüche

1. Verfahren zur Herstellung von Harnstoffen, die von einer α,ω-Diaminosäure abgeleitet sind, gemäß dem man in basischem Medium eine Verbindung, die eine freie Aminogruppe enthält, mit einem Diaminosäurederivat, das eine N^{ω}-Aryloxycarbonylgruppe umfaßt, umsetzt.

2. Verfahren gemäß Anspruch 1, dadurch gekennzeichnet, daß das verwendete Diaminosäurederivat als Aryloxycarbonylgruppe eine Gruppe mit 7 bis 15 Kohlenstoffatomen umfaßt.

3. Verfahren gemäß Anspruch 2, dadurch gekennzeichnet, daß die Aryloxycarbonylgruppe eine Phenyloxycarbonyl- oder Naphthyloxycarbonylgruppe ist, die gegebenenfalls durch wenigstens eine Gruppe substituiert ist, die unter den 1 bis 4 Kohlenstoffatome umfassenden Alkylgruppen und der Nitrogruppe ausgewählt ist.

4. Verfahren gemäß Anspruch 3, dadurch gekennzeichnet, daß die Aryloxycarbonylgruppe die Phenyloxycarbonylgruppe ist.

5. Verfahren gemäß einem der Ansprüche 1 bis 4, dadurch gekennzeichnet, daß die Verbindung, die eine freie Aminogruppe umfaßt, unter Ammoniak, den primären und sekundären Aminen und den Aminosäuren ausgewählt ist.

6. Verfahren gemäß Anspruch 5, dadurch gekennzeichnet, daß die Verbindung, die eine freie Aminogruppe umfaßt, eine Aminosäure ist.

7. Verfahren gemäß Anspruch 6, angewendet auf die Herstellung von N^{ω}-(Aminosäurecarbonyl)- α,ω-diaminosäuren der allgemeinen Formel 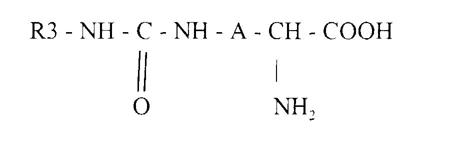 in der A eine zweiwertige Gruppe darstellt, die aus einer von 4 bis 8 Kohlenstoffatomen gebildeten linearen Kohlenstoffkette besteht, die gegebenenfalls durch eine oder mehrere Gruppen, die unter den C₁-C₃-Alkylgruppen und den funktionellen Gruppen, die wenigstens ein Sauerstoff- oder Schwefelatom umfassen, wie eine Carboxyl-, Acyl-, Hydroxy-, Alkoxy- oder Mercaptogruppe, ausgewählt sind, substituiert ist, und in der R₃-NH eine Aminosäure oder ein Peptid darstellt.

8. Verfahren gemäß Anspruch 6, angewendet auf die Herstellung von cyclischen Harnstoffen der allgemeinen Formel 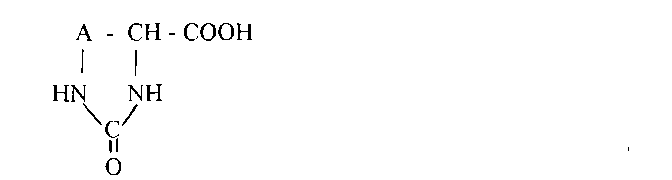 in der A eine zweiwertige Gruppe darstellt, die aus einer von 1 bis 3 Kohlenstoffatomen gebildeten linearen Kohlenstoffkette besteht, die gegebenenfalls durch eine oder mehrere Gruppen, die unter den C₁-C₃-Alkylgruppen und den funktionellen Gruppen, die wenigstens ein Sauerstoff- oder Schwefelatom umfassen, wie eine Carboxyl-, Acyl-, Hydroxy-, Alkoxy- oder Mercaptogruppe, ausgewählt sind, substituiert ist, mit Ausnahme der 2-Oxo-imidazolidin-4-carbonsäure und der (LD)-2-Oxo-hexahydropyrimidin-4-carbonsäure.

9. Verfahren gemäß Anspruch 8, angewendet auf die Herstellung eines cyclischen Harnstoffs, in dem A eine Trimethylengruppe -(CH₂)₃- darstellt.
